# EUROPEAN PATENT SPECIFICATION

(11) **EP 4 110 245 B1**
(45) Date of publication and mention of the grant of the patent: **29.04.2026**
(21) Application number: 21706767.7
(22) Date of filing: 25.01.2021
(51) Int. Cl.: A61F 5/44, A61F 5/451, A61M 25/00

(54) **SEGMENTED DRAINAGE TUBES**
SEGMENTIERTE DRAINAGEROHRE
TUBES SEGMENTÉS DE DRAINAGE

(30) Priority: 28.02.2020 US 202062983414 P
(43) Date of publication of application: 04.01.2023
(73) Proprietor: C. R. Bard, Inc., Franklin Lakes, NJ 07417 (US)
(72) Inventor: TOURCHAK, Michelle, Atlanta, GA 30309 (US); JOHANNES, Ashley, Atlanta, GA 30340 (US)
(74) Representative: Hoffmann Eitle
(86) International application number: PCT/US2021/014935
(87) International publication number: WO 2021/173275

(56) References cited:
- WO-A1-2007/122174
- WO-A1-2018/075464
- US-A1- 2007 225 688

## Description

### BACKGROUND

WO 2007/122174 A1 relates to a dry chamber for a modular drainage system, the dry chamber which may comprise a downstream connector which is in fluid connection with the dry chamber via a flexible annular element defining a conduit.
US 2007/225688 A1 relates to a urinary catheter device which improves on prior devices by providing for a means to lengthen and shorten the tube used to transport urine to a collection receptacle.
WO 2018/075464 A1 relates to urinary catheter assemblies with pre-attached urine collection containers.

### SUMMARY

Briefly summarized, embodiments disclosed herein are directed to systems and methods of drainage tubes displaying differing mechanical properties along an axial length. These drainage tubes can be provided as integrally formed tubes or as modular systems and can be provided as part of a kit. The differing mechanical properties allows a user to position the tube and to prevent the formation of dependent loops, which allow fluid to pool therein.

Dependent loops within drainage tubes can cause fluid to pool, or result in retrograde flow, within drainage tubes. Fluid pooling or retrograde flow can cause various complications. For example, urine pooling can be a source of catheter associated urinary tract infection ("CAUTI") causing agents such as bacteria, microbes, and the like. Hospital Acquired Infections ("HAI"), such as CAUTI, are detrimental to the patient, and also incur extra costs in treating these additional complications.

A fluid drainage system, including, a catheter, a container, and a tube providing fluid communication between the catheter and the container. The tube including a container connector, a proximal mid-section, a distal mid-section, and a catheter connector. Wherein the container connector and the proximal mid-section include one of a first material that displays rigid properties or a second material that displays malleable properties, and the distal mid-section and the catheter connector include one of a second material that displays malleable properties or a third material that displays elastic properties.

In some embodiments, wherein the first material includes one of a plastic, polymer, high density polyethylene (HOPE), nylon, metal, or alloy. The second material includes one of a plastic, polymer, elastomer, metal, alloy, or Nitinol. The third material includes one of a plastic, polymer, elastomer, Polyvinyl chloride (PVC), silicone, or rubber. The container connector provides fluid communication between the container and the proximal mid-section, the proximal mid-section provides fluid communication between the container connector and the distal mid-section, the distal mid-section provides fluid communication between the proximal mid-section and the catheter connector, and the catheter connector provides fluid communication between the distal mid-section and the catheter. One or more of the portions of the tube are coupled using one of welding, bonding, or adhesive.

Also disclosed is a drainage kit, including one of a catheter or a collection container, and a modular drainage tube, including a container connector section, a proximal mid-section, a distal mid-section, and a catheter connector section, wherein each of the container connector section and the proximal mid-section include one of a first material that displays rigid properties, a second material that displays malleable properties, or a third material that displays elastic properties, and each of the distal mid-section and the catheter connector section include one of a second material that displays malleable properties or a third material that displays elastic properties.

In some embodiments, the first material includes one of a plastic, polymer, high density polyethylene (HDPE), nylon, metal, or alloy. The second material includes one of a plastic, polymer, elastomer, metal, alloy, or Nitinol. The third material includes one of a plastic, polymer, elastomer, Polyvinyl chloride (PVC), silicone, or rubber. Each of the container connector section, the proximal mid-section, the distal mid-section, and the catheter connector section include a coupling device configured to couple with a corresponding coupling device and provide fluid communication therebetween. An axis of a distal end of the container connector section is angled relative to an axis of a proximal end of the container connector section at an angle of between 30° and 80°. An axis of a distal end of the catheter connector section is angled relative to an axis of a proximal end of the catheter connector section at an angle of between 30° and 80°.

Also disclosed is a method of draining fluid including, providing a drainage tube including, a malleable container connector section, a rigid proximal mid-section, an elastic distal mid-section, and an elastic catheter connector section, coupling a proximal end of the malleable container connector to a container to provide fluid communication therebetween, coupling a distal end of the elastic catheter connector to a catheter to provide fluid communication therebetween, manipulating the malleable container connector to an angle relative to a vertical axis, and positioning the rigid proximal mid-section at a negative incline relative to a proximal fluid flow.

### DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 shows an exemplary catheter and fluid collection system, in accordance with embodiments disclosed herein.
FIG. 2A shows a fluid collection system including an integral drainage tube including differing mechanical properties, in accordance with embodiments disclosed herein.
FIG. 2B is an enlarged view of a distal end of the drainage tube, in accordance with embodiments disclosed herein.
FIGS. 3A-3C show exemplary configurations of a drainage tube, in accordance with embodiments disclosed herein.
FIG. 4 shows a fluid collection system including a modular drainage tube including differing mechanical properties, in accordance with embodiments disclosed herein.
FIG. 5 shows a kit for a modular drainage tube, in accordance with embodiments disclosed herein.

### DESCRIPTION

Before some particular embodiments are disclosed in greater detail, it should be understood that the particular embodiments disclosed herein do not limit the scope of the concepts provided herein. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the concepts provided herein. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

With respect to "proximal," a "proximal portion" or a "proximal end portion" of, for example, a catheter disclosed herein includes a portion of the catheter intended to be near a clinician when the catheter is used on a patient. Likewise, a "proximal length" of, for example, the catheter includes a length of the catheter intended to be near the clinician when the catheter is used on the patient. A "proximal end" of, for example, the catheter includes an end of the catheter intended to be near the clinician when the catheter is used on the patient. The proximal portion, the proximal end portion, or the proximal length of the catheter can include the proximal end of the catheter; however, the proximal portion, the proximal end portion, or the proximal length of the catheter need not include the proximal end of the catheter. That is, unless context suggests otherwise, the proximal portion, the proximal end portion, or the proximal length of the catheter is not a terminal portion or terminal length of the catheter.

With respect to "distal," a "distal portion" or a "distal end portion" of, for example, a catheter disclosed herein includes a portion of the catheter intended to be near or in a patient when the catheter is used on the patient. Likewise, a "distal length" of, for example, the catheter includes a length of the catheter intended to be near or in the patient when the catheter is used on the patient. A "distal end" of, for example, the catheter includes an end of the catheter intended to be near or in the patient when the catheter is used on the patient. The distal portion, the distal end portion, or the distal length of the catheter can include the distal end of the catheter; however, the distal portion, the distal end portion, or the distal length of the catheter need not include the distal end of the catheter. That is, unless context suggests otherwise, the distal portion, the distal end portion, or the distal length of the catheter is not a terminal portion or terminal length of the catheter.

As used herein, the term "elastic" properties refers to the mechanical properties of deforming when a force is applied and then returning to its original shape when the force is removed. The term "malleable" properties includes deforming, without rupturing, when a force is applied and remaining in the deformed state when the force is removed. The term "rigid" properties refers to the mechanical properties of being resistant to deformation and displaying little or no deformation when a force is applied.

To assist in the description of embodiments described herein, a longitudinal axis extends substantially parallel to an axial length of the tube 120. A lateral axis extends normal to the longitudinal axis, and a transverse axis extends normal to both the longitudinal and lateral axes. Unless defined otherwise, all technical and scientific terms used herein have the same meaning as commonly understood by those of ordinary skill in the art.

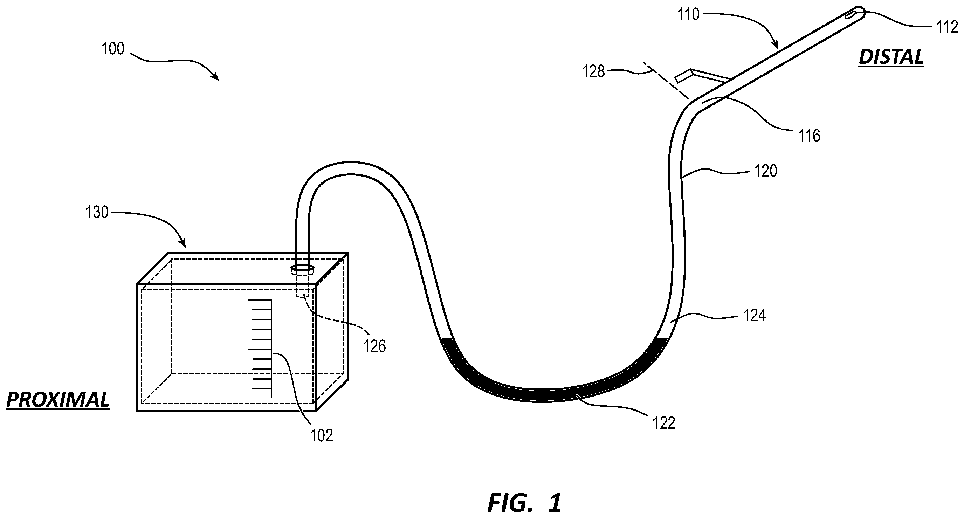

FIG. 1 shows an exemplary fluid collection system 100, which generally includes a catheter 110, a drainage tube ("tube") 120, and a collection container ("container") 130. Exemplary catheters 110 include indwelling catheters, Foley catheters, balloon catheters, peritoneal drainage catheters, or the like, and are configured to be inserted into an orifice within the body of a patient to drain a fluid therefrom. In an embodiment, the catheter 110 can be inserted through the urethra and into a bladder of a patient. The catheter 110 includes an eyelet 112 that provides fluid communication with a lumen of the catheter 110, and is configured to drain a fluid, e.g. urine.

The tube 120 extends from a distal end 128 to a proximal end 126 to define an axial length, and defines a lumen 124. The distal end 128 of the tube 120 is configured to engage a proximal 116 end of the catheter 110. The tube 120 provides fluid communication between the lumen of the catheter 110 and the collection container 130. The tube 120 can be formed of rubber, plastic, polymer, silicone, or similar compliant material. The collection container 130 can include a rigid container, a flexible collection bag, or similar suitable container for receiving a fluid, e.g. urine, drained from the catheter 110. In an embodiment, the container 130 includes graduated markings 102 for measuring a fluid disposed therein.

As shown in FIG. 1, the elasticity of the drainage tube 120 allows dependent loops 122 to form, which can lead to urine pooling within the tube 120. Urine pooling within the tube 120 can be a source of CAUTI causing agents, e.g. microbes, bacteria, etc.

Embodiments disclosed herein include a tube displaying different mechanical properties along an axial length thereof. For example, different portions of the tube can display different mechanical properties including rigid, malleable, or elastic. The rigid or malleable portions of the tube allows a user to position tube and to provide a consistent negative incline, relative to the desired flow direction. This prevents the formation of dependent loops and prevents fluid pooling within the tube. The different portions of the tube can be formed integrally with the tube, or formed separately and connected together in a modular system to suit the user's needs, as described in more detail herein. In an embodiment, the integrated tube 220 or the modular tube 320 can be provided as part of a kit including a catheter 110, collection container 130, and various other equipment such as gloves, lubricant, disinfectant wipes, hand sanitizer, or combinations thereof, and the like.

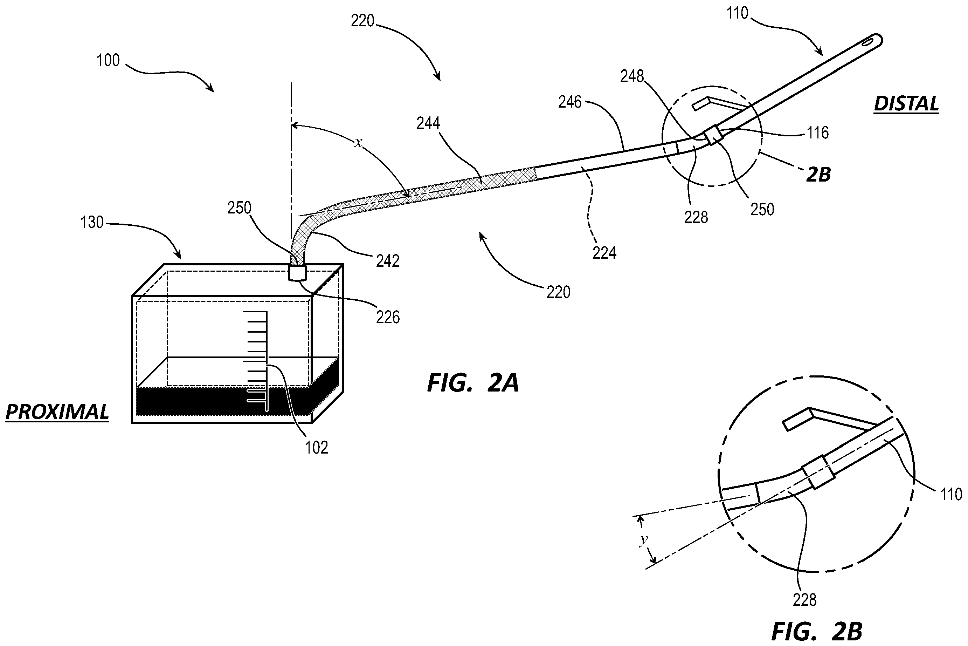

FIG. 2A shows a fluid collection system 100 including a tube 220 extending from a proximal end 226 to a distal end 228 and defines a lumen 224 that provides fluid communication between a catheter 110, coupled to the distal end 228, and a collection container 130 coupled to a proximal end 226. In an embodiment, the tube 220 includes one or more portions formed integrally as a single structure, and where each portion can provide different mechanical properties including rigid, malleable, or elastic. In an embodiment, the one or more portions can be coupled by welding, bonding, adhesive, or the like.

As shown in FIG. 2A, the tube 220 includes one or more portions including a container connector 242, a proximal mid-section 244, a distal mid-section 246, and a catheter connector 248. The container connector 242 extends from the proximal end 226 of the tube 220 to a proximal end of the proximal mid-section 244. The proximal mid-section 244 extends from a distal end of the container connector 242 to a proximal end of the distal mid-section 246. The distal mid-section 246 extends from a distal end of the proximal mid-section 244 to a proximal end of the catheter connector 248. The catheter connector 248 extends from a distal end of the distal mid-section 246 to a distal end 228 of the tube 220.

The container connector 242 can be coupled, and in fluid communication with, the container 130 using a coupling device "coupler" 250 such as a luer slip, luer lock, spin nut, twist lock, or similar suitable connector to provide fluid communication between the container connector portion 242 and the container 130. The catheter connector portion 248 can be fluidly coupled with the catheter 110 using a coupling device "coupler" 250 such as luer slip, luer lock, spin nut, twist lock, or similar suitable connector to provide fluid communication between the catheter connector portion 248 and the catheter 110.

As described in more detail herein, each of the container connector portion 242, the proximal mid-section 244, the distal mid-section 246, and the catheter connector portion 248 can include one of a first material, a second material, or a third material. In an embodiment, a first material can display "rigid" mechanical properties and can include plastic, polymer, high density polyethylene (HDPE), nylon, metal, alloy, combinations thereof, or the like. In an embodiment, a second material can display "malleable" properties and can include plastic, polymer, elastomer, metal, alloy, Nitinol, combinations thereof, or the like. In an embodiment, a third material can display "elastic" properties and can include plastic, polymer, elastomer, Polyvinyl chloride (PVC), silicone, rubber, combinations thereof, or the like.

In an embodiment, the container connector 242 can be formed of a rigid material and can define an arcuate shape such that a distal end of the container connector 242 is angled (*x*) from a vertical axis of between 0° and 90°. In an embodiment, the container connector 242 is angled (*x*) from a vertical axis of between 30° and 80°. As described herein, the container connector 242, formed of a rigid material, can resist a deformation when a force is applied. Advantageously, the angled, rigid container connector 242 maintains the tube 220 at a consistent negative incline relative to a proximal direction of flow. This prevents urine from pooling within the tube 220, or from forming dependent loops.

In an embodiment, the container connector 242 formed of a malleable material. As such a user can position the container connector 242 such that at distal end is angled from a vertical axis and remain in position until it is repositioned. Advantageously, the positionable, malleable, container connector 242 can be positioned to a desired angle, and can remain at that position until repositioned, to maintain the tube 220 at a consistent negative incline relative to a proximal direction of flow. This prevents urine from pooling within the tube 220, or from forming dependent loops. In an embodiment, the container connector 242 formed of an elastic material that can flex according to the position of the mid-sections 244, 246. Advantageously, the container connector 242 can flex to accommodate the position of the tube 220.

In an embodiment, the proximal mid-section 244 can be formed of a rigid material and can define substantially straight fluid pathway extending between the distal mid-section 246 and the container connector 242. As described herein, the proximal mid-section 244, formed of a rigid material, can resist a deformation when a force is applied. Advantageously, the straight, rigid proximal mid-section 244 can be positioned to maintain a consistent negative incline relative to a proximal direction of flow. This prevents urine from pooling within the tube 220, or from forming dependent loops. In an embodiment, the straight, rigid proximal mid-section 244 can be connected to the angled, rigid container connector 242. The angled, rigid container connector 242 maintains the rigid proximal mid-section 244 at a consistent negative incline and the rigid proximal mid-section 244 prevents dependent loops from forming. In an embodiment, the straight, rigid proximal mid-section 244 can be connected to a malleable container connector 242. The malleable container connector 242 can be positioned at an angle relative to a vertical axis and maintains the rigid proximal mid-section 242 at a consistent negative incline until it is repositioned. Further, the rigid proximal mid-section 242 prevents dependent loops from forming.

In an embodiment, the proximal mid-section 244 can be formed of a malleable material. As such a user can position the proximal mid-section 244 to suit a particular path between the catheter 110 and the container 130, as well as maintain a consistent negative incline therebetween. The positionable, malleable, proximal mid-section 244 can be positioned to the particular path, and can remain at that position until repositioned. Advantageously, the malleable proximal mid-section 244 can be shaped to define a path and negotiate different furniture that maybe in between the catheter 110 and the container 130, e.g. hospital bed, wheel chair, intravenous stands or poles, or the like. Advantageously, the malleable proximal mid-section 244 can be shaped to hook onto or wrap around different objects or furniture, securing the tube thereto. As used herein, the malleable portions of tube allow a user to shape the path of the tube while maintaining the patency of the tube.

In an embodiment, the distal mid-section 246 can be formed of a rigid material and can define substantially straight fluid pathway extending between the catheter connector 248 and the proximal mid-section 244. As described herein, the distal mid-section 246, formed of a rigid material, can resist a deformation when a force is applied. Advantageously, the straight, rigid distal mid-section 246 can be connected to the proximal mid-section 244 which in turn can be positioned at a consistent negative incline as described herein. The straight, rigid distal mid-section 246 prevents urine from pooling within the tube 220, or from forming dependent loops. In an embodiment, the straight, rigid distal mid-section 246 can be coupled with an elastic catheter connector 248, as described herein. This allows the catheter 110 to flex relative to the mid-sections 244, 246, and allows the patient to move comfortably without repositioning the mid-sections 244, 246.

In an embodiment, the distal mid-section 244 can be formed of a malleable material. As such a user can position the distal mid-section 244 to suit a particular path between the catheter 110 and proximal mid-section 244, and/or maintain a consistent negative incline therebetween. The positionable, malleable, distal mid-section 246 can be positioned to the particular path, and can remain at that position until repositioned, Advantageously, the malleable proximal mid-section 244 can be shaped to define a path and negotiate different furniture that maybe in between the catheter 110 and the proximal mid-section 244, e.g. hospital bed, wheel chair, intravenous stands or poles, or the like. For example, a tube 220 can include a rigid container connector 242, a rigid proximal mid-section 244 to define a consistent negative incline. The malleable distal mid-section 246 can then be positioned to define a path along a surface of a hospital bed to a groin region to connect with a flexible catheter connector 248 and catheter 110. In an embodiment, the distal mid-section 244 can be formed of an elastic material. Advantageously, the distal mid-section 244 can flex to suit the movement of the patient without disrupting the position of the proximal mid-section 244, or the container connector 242.

In an embodiment, the catheter connector 248 can be formed of an elastic material. Advantageously, the catheter connector 248 can flex to suit the movement of the patient without disrupting the position of the mid-sections 244, 246, or container connector 242. In an embodiment, the catheter connector 248 can be formed of a malleable material and can be positioned to a preferred angle, relative to a longitudinal axis of the catheter 110. Advantageously, the catheter connector 248 can be positionable to a position and remain in that position until repositioned. For example, the catheter connector 248 can be positioned to divert the tube away from a mid-line of the body, towards a left or right side.

In an embodiment, the catheter connector 248 can be formed of rigid material and define a substantially straight shape. As shown in FIG. 2A, for example, the catheter connector 248 can define an arcuate shape such that a proximal end of the container connector 242 is angled (y) from a longitudinal axis of the catheter 110 of between 0° and 90°. In an embodiment, the container connector 242 is angled (y) from a longitudinal axis of between 5° and 15°. As described herein, the container connector 242, formed of a rigid material, can resist a deformation when a force is applied. Advantageously, the angled, rigid container connector 242 maintains the tube 220 at a consistent negative incline relative to a proximal direction of flow. This prevents urine from pooling within the tube 220, or from forming dependent loops.

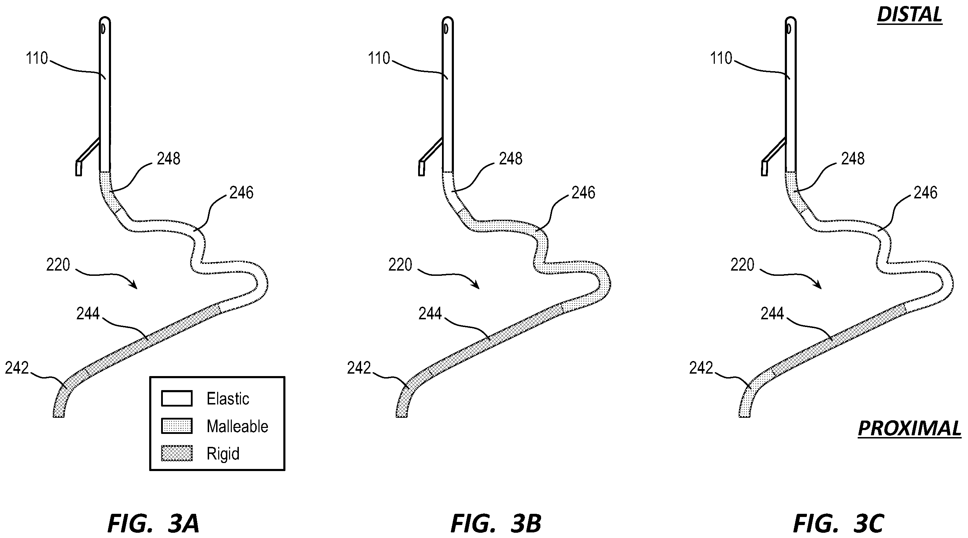

FIGS. 3A-3C show exemplary configurations of the integrally formed tube 220. It will be appreciated that the configurations shown can also apply to a modular tube system, as described herein. As shown in FIG. 3A, the container connector 242 and the proximal mid-section 244 are formed of a rigid material, the distal mid-section 246 is formed of an elastic material, and the catheter connector 248 is formed of a malleable material. Advantageously, the rigid sections are disposed towards a proximal end, which is typically situated lower than the rest of the tube and the catheter 110. These rigid sections maintain a negative incline through the proximal part of the tube and maintains a through-flow to the container, preventing urine pooling. The malleable catheter connector 248 allows a user to position the portion of tube leading immediately away from the catheter, at a comfortable angle, for example off to one side. The elastic distal mid-section 246 allows a degree of movement between the catheter 110 and the proximal mid-section 244. This allows the user to move comfortably without disrupting the position of the rigid portions of the tube 220.

As shown in FIG. 3B, the container connector 242 and the proximal mid-section 244 are formed of a rigid material, the distal mid-section 246 is formed of a malleable material, and the catheter connector 248 is formed of an elastic material. The rigid sections maintain a negative incline through the proximal part of the tube, as described herein. The elastic catheter connector 248 allows a degree of movement between the catheter 110 and the distal mid-section 246 and allows the user to move comfortably. The malleable distal mid-section 246 allows a user to position the portion of tube leading away from the catheter in a comfortable position, and for the distal mid-section 246 to remain in the position until repositioned. For example, the malleable distal mid-section 246 can lead from the catheter to a side edge of a hospital bed where it connects with the rigid portions of the tube. Advantageously, the user can position the distal mid-section 246 to suit a particular pathway across the bed, and to remain in place once positioned as such. By contrast, an elastic portion might not remain in place and could fall and pull on the catheter, leading to discomfort.

As shown in FIG. 3C, the container connector 242 includes a malleable portion, the proximal mid-section 244 are formed of a rigid material, the distal mid-section 246 is formed of an elastic material, and the catheter connector 248 is formed of a malleable material. Advantageously, the catheter connector 248 can be positioned at a comfortable angle, as described herein. Further the container connector 242 can be positioned to maintain the rigid proximal mid-section 244, connected thereto, at a preferred angle and prevent dependent loops from forming. Further the elastic distal mid-section 246 allows the user to move comfortably. Advantageously, the malleable portions of the tube are configured to deflect under less force than would be required to dislodge the catheter 110 from the body of the patient. Accordingly, while the malleable portions maintain the positioned shape, if the user moves too far from the container 130, the malleable portions are still able to deform and prevent the catheter 110 from dislodging and causing discomfort. It will be appreciated that various other combinations of sections 242, 244, 246, 248 including rigid, malleable, elastic mechanical properties are also contemplated and fall within the scope of the present invention.

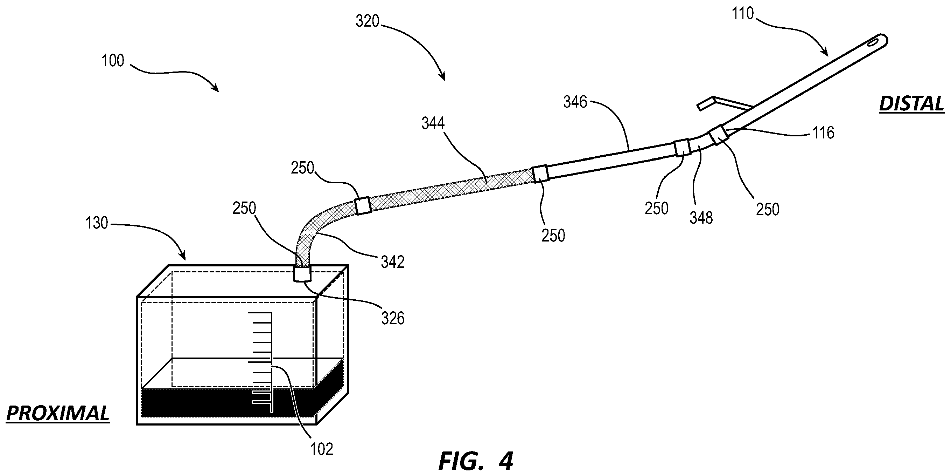

As shown in FIGS. 4-5, in an embodiment, a fluid collection system 100 is provided including a modular drainage tube 320 that includes separate structures of a container connector 342, a proximal mid-section 344, a distal mid-section 346, and a catheter connector 348. Each of the sections 342, 344, 346, 348 include a connector 350 disposed at a distal end and at a proximal end. The couplers 250 are configured to engage a connector of an adjacent section 342, 344, 346, 348, to provide fluid communication therebetween. Exemplary connectors include luer slip, luer locks, spin nuts, or the like, as described herein. Advantageously, a user can select different sections and different mechanical properties to suit the needs of the individual. Further the user can adjust the length of the tube by adding or removing different number of sections. In an embodiment, one or more of the sections 342, 344, 346, 348 can define a straight shape. In an embodiment, one or more of the sections 342, 344, 346, 348 can define an arcuate shape of between 0° and 90°, as described herein.

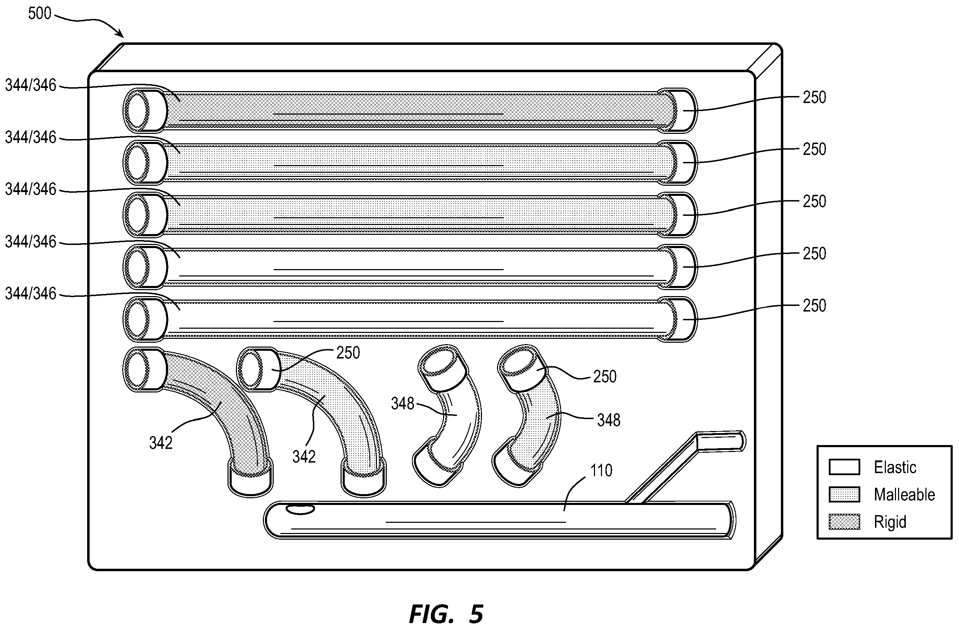

As shown in FIG. 5, a kit 500 is provided including one or more sections of a modular tube 320, such as one or more of a container connector 342, a proximal mid-section 344, a distal mid-section 346, and a catheter connector 348, or combinations thereof. Further the kit can include a catheter 110, container (not shown), and various other equipment (e.g. catheter placement equipment, and the like), as described herein. Advantageously, a user can select different sections of the tube 320 depending on the type of mechanical properties required. Further a user can select a different number of sections depending on the overall length of the tube 320 required. As such, a user can "build" a tube and arrange as necessary to prevent dependent loops from forming.

In an embodiment, the couplers 250 can further include a valve that inhibits fluid flow when the coupler 250 is detached and is opened to allow fluid flow when the connector is attached. Advantageously, this allows a user to switch out different portions of the tube without having to drain the fluid collection system 100, or compromise the sterility of the fluid collection system 100.

## Claims

1. A fluid drainage system, comprising:
a catheter (110);
a container (130); and
a tube (220) providing fluid communication between the catheter (110) and the container (130), the tube (220) comprising:
a container connector (242);
a proximal mid-section (244);
a distal mid-section (246); and
a catheter connector (248);
wherein:
the container connector (242) and the proximal mid-section (244) include one of a first material that displays rigid properties or a second material that displays malleable properties;
the distal mid-section (246) and the catheter connector (248) include one of a second material that displays malleable properties or a third material that displays elastic properties;
**characterised in that**
the first material includes one of a plastic, polymer, high density polyethylene (HDPE), nylon, metal, or alloy;
the second material includes one of a plastic, polymer, elastomer, metal, alloy, or Nitinol; and
the third material includes one of a plastic, polymer, elastomer, Polyvinyl chloride (PVC), silicone, or rubber.

2. The fluid drainage system of claim 1, wherein the container connector (242) provides fluid communication between the container (130) and the proximal mid-section (244), the proximal mid-section (244) provides fluid communication between the container connector (248) and the distal mid-section (246), the distal mid-section (246) provides fluid communication between the proximal mid-section (244) and the catheter connector (248), and the catheter connector (248) provides fluid communication between the distal mid-section (246) and the catheter (110).

3. The fluid drainage system of either claim 1 or claim 2, wherein one or more of the portions of the tube (220) are coupled using one of welding, bonding, or adhesive.

4. A drainage kit, comprising:
one of a catheter (110) or a collection container (242); and
a modular drainage tube (320), comprising:
a container connector section (342);
a proximal mid-section (344);
a distal mid-section (346); and
a catheter connector section (348);
wherein:
each of the container connector section (342) and the proximal mid-section (344) include one of a first material that displays rigid properties, a second material that displays malleable properties, or a third material that displays elastic properties;
each of the distal mid-section (346) and the catheter connector section (348) include one of a second material that displays malleable properties or a third material that displays elastic properties;
**characterised in that**
the first material includes one of a plastic, polymer, high density polyethylene (HDPE), nylon, metal, or alloy;
the second material includes one of a plastic, polymer, elastomer, metal, alloy, or Nitinol; and
the third material includes one of a plastic, polymer, elastomer, Polyvinyl chloride (PVC), silicone, or rubber.

5. The drainage kit according to claim 4, wherein each of the container connector section (342), the proximal mid-section (344), the distal mid-section (346), and the catheter connector section (348) include a coupling device (250) configured to couple with a corresponding coupling device (250) and provide fluid communication therebetween.

6. The drainage kit according to either claim 4 or claim 5, wherein an axis of a distal end of the container connector section (342) is angled relative to an axis of a proximal end of the container connector section (342) at an angle of between 30° and 80°, and/or wherein an axis of a distal end of the catheter connector section (348) is angled relative to an axis of a proximal end of the catheter connector section (348) at an angle of between 30° and 80°.

7. A method of draining fluid, comprising:
providing a drainage tube (120) comprising:
a malleable container connector section (242, 342),
a rigid proximal mid-section (244, 344),
an elastic distal mid-section (246, 346); and
an elastic catheter connector section (248, 348);
coupling a proximal end of the malleable container connector (242, 342) to a container (130) to provide fluid communication therebetween;
coupling a distal end of the elastic catheter connector (248, 348) to a catheter (110) to provide fluid communication therebetween;
manipulating the malleable container connector (242, 342) to an angle relative to a vertical axis; and
positioning the rigid proximal mid-section (244, 344) at a negative incline relative to a proximal fluid flow,
**characterised in that**
the malleable container connector (242, 342) is formed of one of a plastic, polymer, or elastomer,
**in that** the rigid proximal mid-section (244, 344) is formed of one of a plastic, polymer, high density polyethylene (HDPE), nylon, metal, or alloy, and
**in that** one of the elastic distal mid-section (246, 346) or the elastic catheter connector section (248, 348) is formed of one of a plastic, polymer, elastomer, Polyvinyl chloride (PVC), silicone, or rubber.

8. The method according to claim 7, wherein the one or more sections are coupled to each other using a fluid connection device and/or by welding, bonding, or adhesive.

## Patentansprüche

1. Fluiddrainagesystem, umfassend:
einen Katheter (110);
einen Behälter (130); und
ein Rohr (220), das eine Fluidverbindung zwischen dem Katheter (110) und dem Behälter (130) bereitstellt, wobei das Rohr (220) umfasst:
einen Behälterverbinder (242);
einen proximalen Mittelabschnitt (244);
einen distalen Mittelabschnitt (246); und
einen Katheterverbinder (248);
wobei:
der Behälterverbinder (242) und der proximale Mittelabschnitt (244) eines von einem ersten Material, das starre Eigenschaften aufweist, oder einem zweiten Material, das verformbare Eigenschaften aufweist, einschließen;
der distale Mittelabschnitt (246) und der Katheterverbinder (248) eines von einem zweiten Material, das verformbare Eigenschaften aufweist, oder einem dritten Material, das elastische Eigenschaften aufweist, einschließen;
**dadurch gekennzeichnet, dass**
das erste Material eines von einem Kunststoff, einem Polymer, Polyethylen hoher Dichte (HDPE), Nylon, Metall oder einer Legierung einschließt;
das zweite Material eines von einem Kunststoff, einem Polymer, einem Elastomer, einem Metall, einer Legierung oder Nitinol einschließt; und
das dritte Material eines von einem Kunststoff, einem Polymer, einem Elastomer, Polyvinylchlorid (PVC), Silikon oder Gummi einschließt.

2. Fluiddrainagesystem nach Anspruch 1, wobei der Behälterverbinder (242) eine Fluidverbindung zwischen dem Behälter (130) und dem proximalen Mittelabschnitt (244) bereitstellt, der proximale Mittelabschnitt (244) eine Fluidverbindung zwischen dem Behälterverbinder (248) und dem distalen Mittelabschnitt (246) bereitstellt, der distale Mittelabschnitt (246) eine Fluidverbindung zwischen dem proximalen Mittelabschnitt (244) und dem Katheterverbinder (248) bereitstellt, und der Katheterverbinder (248) eine Fluidverbindung zwischen dem distalen Mittelabschnitt (246) und dem Katheter (110) bereitstellt.

3. Fluiddrainagesystem nach Anspruch 1 oder Anspruch 2, wobei ein oder mehrere der Rohrabschnitte des Rohrs (220) unter Verwendung eines von Schweißen, Bonden oder Klebstoff gekoppelt sind.

4. Drainagesatz, umfassend:
eines von einem Katheter (110) oder einem Sammelbehälter (242); und
ein modulares Drainagerohr (320), umfassend:
einen Behälterverbinderabschnitt (342);
einen proximalen Mittelabschnitt (344);
einen distalen Mittelabschnitt (346); und
einen Katheterverbinderabschnitt (348);
wobei:
jeder von dem Behälterverbinderabschnitt (342) und dem proximalen Mittelabschnitt (344) eines von einem ersten Material, das starre Eigenschaften aufweist, einem zweiten Material, das verformbare Eigenschaften aufweist, oder einem dritten Material, das elastische Eigenschaften aufweist, einschließt;
jeder von dem distalen Mittelabschnitt (346) und dem Katheterverbinderabschnitt (348) eines von einem zweiten Material, das verformbare Eigenschaften aufweist, oder einem dritten Material, das elastische Eigenschaften aufweist, einschließt;
**dadurch gekennzeichnet, dass**
das erste Material eines von einem Kunststoff, einem Polymer, Polyethylen hoher Dichte (HDPE), Nylon, Metall oder einer Legierung einschließt;
das zweite Material eines von einem Kunststoff, einem Polymer, einem Elastomer, einem Metall, einer Legierung oder Nitinol einschließt; und
das dritte Material eines von einem Kunststoff, einem Polymer, einem Elastomer, Polyvinylchlorid (PVC), Silikon oder Gummi einschließt.

5. Drainagesatz nach Anspruch 4, wobei jeder von dem Behälterverbinderabschnitt (342), dem proximalen Mittelabschnitt (344), dem distalen Mittelabschnitt (346) und dem Katheterverbinderabschnitt (348) eine Kupplungsvorrichtung (250) einschließt, die ausgebildet ist, mit einer entsprechenden Kupplungsvorrichtung (250) zu koppeln und dortzwischen eine Fluidverbindung bereitzustellen.

6. Drainagesatz nach Anspruch 4 oder Anspruch 5, wobei eine Achse eines distalen Endes des Behälterverbinderabschnitts (342) relativ zu einer Achse eines proximalen Endes des Behälterverbinderabschnitts (342) um einen Winkel von zwischen 30° und 80° angewinkelt ist, und/oder wobei eine Achse eines distalen Endes des Katheterverbinderabschnitts (348) relativ zu einer Achse eines proximalen Endes des Katheterverbinderabschnitts (348) um einen Winkel von zwischen 30° und 80° angewinkelt ist.

7. Verfahren zum Drainieren von Fluid, umfassend:
Bereitstellen eines Drainagerohrs (120) umfassend:
einen verformbaren Behälterverbinderabschnitt (242, 342),
einen starren proximalen Mittelabschnitt (244, 344),
einen elastischen distalen Mittelabschnitt (246, 346); und
einen elastischen Katheterverbinderabschnitt (248, 348);
Koppeln eines proximalen Endes des verformbaren Behälterverbinders (242, 342) an einen Behälter (130), um dortzwischen eine Fluidverbindung bereitzustellen;
Koppeln eines distalen Endes des elastischen Katheterverbinders (248, 348) an einen Katheter (110), um dortzwischen eine Fluidverbindung bereitzustellen;
Manipulieren des verformbaren Behälterverbinders (242, 342) zu einem Winkel relativ zu einer vertikalen Achse; und
Positionieren des starren proximalen Mittelabschnitts (244, 344) bei einer negativen Neigung relativ zu einem proximalen Fluidfluss,
**dadurch gekennzeichnet, dass**
der verformbare Behälterverbinder (242, 342) aus einem von einem Kunststoff, einem Polymer oder einem Elastomer gebildet ist, **dass**
der starre proximale Mittelabschnitt (244, 344) aus einem von einem Kunststoff, einem Polymer, Polyethylen hoher Dichte (HDPE), Nylon, Metall oder einer Legierung gebildet ist, und **dass**
entweder der elastische distale Mittelabschnitt (246, 346) oder der elastische Katheterverbinderabschnitt (248, 348) aus einem von einem Kunststoff, einem Polymer, einem Elastomer, Polyvinylchlorid (PVC), Silikon oder Gummi gebildet ist.

8. Verfahren nach Anspruch 7, wobei der eine oder die mehreren Abschnitte unter Verwendung einer Fluidverbindungsvorrichtung und/oder durch Schweißen, Bonden oder Klebstoff miteinander gekoppelt sind.

## Revendications

1. Système de drainage de fluide, comprenant :
un cathéter (110) ;
un récipient (130) ; et
un tube (220) fournissant une communication fluidique entre le cathéter (110) et le récipient (130), le tube (220) comprenant :
un raccord (242) de récipient ;
une section médiane proximale (244) ;
une section médiane distale (246) ; et
un raccord (248) de cathéter ;
dans lequel :
le raccord (242) de récipient et la section médiane proximale (244) incluent l'un parmi un premier matériau qui présente des propriétés rigides ou un deuxième matériau qui présente des propriétés malléables ;
la section médiane distale (246) et le raccord (248) de cathéter incluent l'un parmi un deuxième matériau qui présente des propriétés malléables ou un troisième matériau qui présente des propriétés élastiques ;
**caractérisé en ce que**
le premier matériau inclut l'un parmi un plastique, un polymère, du polyéthylène haute densité (HDPE), du nylon, un métal ou un alliage ;
le deuxième matériau inclut l'un parmi un plastique, un polymère, un élastomère, un métal, un alliage ou du Nitinol ; et
le troisième matériau inclut l'un parmi un plastique, un polymère, un élastomère, du polychlorure de vinyle (PVC), du silicone ou du caoutchouc.

2. Système de drainage de fluide selon la revendication 1, dans lequel le raccord (242) de récipient fournit une communication fluidique entre le récipient (130) et la section médiane proximale (244), la section médiane proximale (244) fournit une communication fluidique entre le raccord (248) de récipient et la section médiane distale (246), la section médiane distale (246) fournit une communication fluidique entre la section médiane proximale (244) et le raccord (248) de cathéter, et le connecteur (248) de cathéter fournit une communication fluidique entre la section médiane distale (246) et le cathéter (110).

3. Système de drainage de fluide selon l'une ou l'autre de la revendication 1 ou de la revendication 2, dans lequel une ou plusieurs des portions du tube (220) sont couplées en utilisant l'un parmi le soudage, le collage ou l'adhésif.

4. Kit de drainage, comprenant :
l'un parmi un cathéter (110) ou un récipient collecteur (242) ; et
un tube de drainage modulaire (320), comprenant :
une section raccord (342) de récipient ;
une section médiane proximale (344) ;
une section médiane distale (346) ; et
une section raccord (348) de cathéter ;
dans lequel :
chacune de la section raccord (342) de récipient et de la section médiane proximale (344) inclut l'un parmi un premier matériau qui présente des propriétés rigides, un deuxième matériau qui présente des propriétés malléables ou un troisième matériau qui présente des propriétés élastiques ;
chacune de la section médiane distale (346) et de la section raccord (348) de cathéter inclut l'un parmi un deuxième matériau qui présente des propriétés malléables ou un troisième matériau qui présente des propriétés élastiques ;
**caractérisé en ce que**
le premier matériau inclut l'un parmi un plastique, un polymère, du polyéthylène haute densité (HDPE), du nylon, un métal ou un alliage ;
le deuxième matériau inclut l'un parmi un plastique, un polymère, un élastomère, un métal, un alliage ou du Nitinol ; et
le troisième matériau inclut l'un parmi un plastique, un polymère, un élastomère, du polychlorure de vinyle (PVC), du silicone ou du caoutchouc.

5. Kit de drainage selon la revendication 4, dans lequel chacune de la section raccord (342) de récipient, de la section médiane proximale (344), de la section médiane distale (346) et de la section raccord (348) de cathéter inclut un dispositif de couplage (250) configuré pour se coupler avec un dispositif de couplage (250) correspondant et fournir une communication fluidique entre ceux-ci.

6. Kit de drainage selon l'une ou l'autre de la revendication 4 ou de la revendication 5, dans lequel un axe d'une extrémité distale de la section raccord (342) de récipient est incliné par rapport à un axe d'une extrémité proximale de la section raccord (342) de récipient à un angle compris entre 30° et 80°, et/ou dans lequel un axe d'une extrémité distale de la section raccord (348) de cathéter est incliné par rapport à un axe d'une extrémité proximale de la section raccord (348) de cathéter à un angle compris entre 30° et 80°.

7. Procédé de drainage de fluide, comprenant :
la fourniture d'un tube de drainage (120) comprenant :
une section raccord (242, 342) de récipient malléable,
une section médiane proximale rigide (244, 344),
une section médiane distale élastique (246, 346) ; et
une section raccord (248, 348) de cathéter élastique ;
le couplage d'une extrémité proximale du raccord (242, 342) de récipient malléable à un récipient (130) pour fournir une communication fluidique entre ceux-ci ;
le couplage d'une extrémité distale du raccord (248, 348) de cathéter élastique à un cathéter (110) pour fournir une communication fluidique entre ceux-ci ;
la manipulation du raccord (242, 342) de récipient malléable à un angle par rapport à un axe vertical ; et
le positionnement de la section médiane proximale rigide (244, 344) à une inclinaison négative par rapport à un écoulement de fluide proximal,
**caractérisé en ce que**
le raccord (242, 342) de récipient malléable est formé de l'un parmi un plastique, un polymère ou un élastomère, **en ce que**
la section médiane proximale rigide (244, 344) est formée de l'un parmi un plastique, un polymère, du polyéthylène haute densité (HDPE), du nylon, un métal ou un alliage, et **en ce que**
l'une parmi la section médiane distale élastique (246, 346) ou la section raccord (248, 348) de cathéter élastique est formée de l'un parmi un plastique, un polymère, un élastomère, du polychlorure de vinyle (PVC), du silicone ou du caoutchouc.

8. Procédé selon la revendication 7, dans lequel les une ou plusieurs sections sont couplées les unes aux autres en utilisant un dispositif de raccordement fluidique et/ou par soudage, collage ou adhésif.
